# EUROPEAN PATENT APPLICATION

(11) **EP 4 721 669 A1**
(43) Date of publication of application: **08.04.2026**
(21) Application number: 24204186.1
(22) Date of filing: 02.10.2024
(51) Int. Cl.: A61B 6/03

(54) **ITERATIVE CT SCAN PROTOCOL**

(71) Applicant: Koninklijke Philips N.V., 5656 AG Eindhoven (NL)
(72) Inventor: SCHMITT, Holger, Eindhoven (NL); DESHPANDE, Hrishikesh Narayanrao, Eindhoven (NL); BUELOW, Thomas, 5656AG Eindhoven (NL)
(74) Representative: Philips Intellectual Property & Standards

(57) **Abstract**

System and related method for tomographic imaging. The system may comprise an input interface for receiving a scan request for a region of interest in a surrounding object. Control logic is operable to cause a tomographic imaging apparatus in an imaging session to acquire, along an acquisition axis (Z), multiple projection frames *λ*(*z*) of the surrounding object optionally with application of an image quality, IQ, booster operation, for ranges along the acquisition axis of the imaging apparatus. Whilst the imaging session is ongoing, based on a quality assessment in relation to the acquired projection frames, the control logic is to cause revisit one or more times, an earlier range and re-acquire there fresh projection fames *λ'*(*z*), with application of the image quality, IQ, booster operation, until the quality assessment is indicative of the quality being on target, or until a stopping condition is met.

## Description

### FIELD OF THE INVENTION

The invention relates to a system for tomographic imaging, to a related method, to an imaging arrangement, to a computer program element and to a computer readable medium.

### BACKGROUND OF THE INVENTION

Medical imaging is among the most useful tools in the arsenal of the modem medic. Medical imaging allows obtaining insight into internal structures and tissues of a patient in a non-invasive manner, to thereby inform diagnosis, therapy and planning, etc., whatever the clinical case at hand. X-ray based imaging is among the oldest and most widely used medical imaging techniques, with X-ray based tomographic (CT - computed tomography) reaching beyond the confines of the projection domain, to cross-sectional imaging in 3D space (image domain). CT is in general more low cost as compared to other tomographic imaging modalities, such as MRI (magnetic resonance imaging), and affords great versatility across various medical imaging needs.

The contrast in obtained imagery needs to be satisfactory. A higher signal to noise ratio, that is, good image contrast, is at least in part a function of the X-ray dosage used. X-ray imaging is a double-edged sword and requires carefully balancing of two opposing effects: on the one hand side the beneficial and sought after need for good quality imagery, and on the other hand the harmful effects that X-ray poses for biological tissue. The higher the dose, the more harmful this latter effect is, but, equally, a higher dose may yield better image quality ("IQ"). The use of X-rays in an efficient manner is of consideration. In this context, efficiency may be conceptualized as the amount of medically relevant information that can be gained from the obtained imagery versus the expended dose. This concept may be referred herein as the "efficiency ratio". The more image-based information can be gained/extracted for a given unit of X-ray radiation dose spent, the better the ratio. And this is desirable. Currently, certain imaging protocols (that is, the manner in which X-radiation is expended in an imaging session) are used that may be said to yield comparatively low such efficiency ratio. Among such protocols are surview-based imaging protocols.

Typically, such CT scan protocols include a first low-dose surview scan (2D or 3D), which is used to plan a second higher-dose diagnostic scan. The diagnostic scan usually has a shorter range, and the X-ray dose is set such that diagnostic certainty can be achieved over the field-of-view selected in the surview image. Most scanners vary dose based purely on an estimation of the water-equivalent diameter (WED) of the patient along the scan range in order to avoid overexposure.

It is found herein that the efficiency ratio could be improved, in particular over such surview-based X-ray imaging protocols, more particularly still, over surview-based CT imaging protocols. Thus, it may be one objective herein to address at least some of the above-mentioned deficiencies, in particular, increase the efficiency of X-ray based imaging, in particular in tomography.

### SUMMARY OF THE INVENTION

Thus, an objective of the present invention is achieved by the subject matter of the independent claims, where further embodiments are incorporated in the dependent claims. It should be noted that the following described aspect of the invention equally applies to the related method, the imaging arrangement, to the computer program element and to the computer readable medium.

Specifically, at least some of the above-mentioned deficiencies are addressed herein at least in part with the following proposed system and methods for X-ray imaging, in particular for tomographic X-ray imaging, in particular still, by providing a control system as envisaged herein that administers a new imaging protocol in which, in some embodiments, X-ray dose along the tomographic rotation axis is expended in a non-uniform spatial profile, with such dose being expended differently over different spatial ranges along such axis, based on, or driven by, iterative IQ (image quality) checks.

According to a first aspect of the invention there is provided a system for tomographic imaging, comprising:
- an input interface for receiving a scan request for a region of interest in a surrounding object; and
- control logic/module operable to cause a tomographic imaging apparatus in an imaging session to:
   acquire, along an acquisition axis, multiple projection frames of the surrounding object, optionally with application or re-application of an image quality, IQ, booster operation, for one or more ranges along the acquisition axis of the imaging apparatus; and
   whilst the imaging session is ongoing, based on a quality assessment in relation to the acquired projection frames, revisit one or more times, at least one earlier range and re-acquiring there fresh projection fames, with application of the image quality, IQ, booster operation.

In embodiments, once the quality assessment is on target, the control logic is operable to cause no longer revisiting the relevant range for reacquisition in the current imaging session.

In embodiments, the control logic may cause revisiting, one or more times, for reacquisition of fresh frames at other one or more ranges or same rang(es), with application or re-application of the image quality, IQ, booster operation, if the quality assessment is off target, and to cause the so revisiting and reacquiring other fresh frames there, with respective (e.g., prior to some or each re-acquiring) application of the image quality, IQ, booster operation, until the quality assessment is on target or the stopping condition is met.

In embodiments, the quality assessment is based on respective slice imagery reconstructable by a reconstructor from the projection frames for the respective range along the acquisition axis.

In embodiments, the slice imagery is a single such slice per range or is volumetric.

In embodiments, the at least one or more of the one or more ranges is a point on the axis. Thus, the range as used includes a position of slice along the axis, but may also include a genuine range of set of multiple positions.

In embodiments, the quality assessment includes at least one score that pertains to at least one of: i) an image quality, IQ, score in relation to or based on the respective projection frames per respective range on the axis, ii) a certainty score, IC, in relation to at least one finding based on the respective projection frames per range on the axis.

In embodiments, the quality assessment is based on both, IQ and IC.

In embodiments, the quality assessment is two-stage, based first on IQ and then on IC, wherein the revisiting of some at least one range and reacquisition of fresh projection frames there is done only for the said at least one or more range for which the IQ is off target, or for which the IQ is on target, but the IC is not.

In embodiments, there are plural findings, and wherein one of the findings appertains to the region of interest, and at least one other finding appertains to at least one other subsidiary region located elsewhere in respect to the axis Z from where the region of interest is located.

In embodiments, the revisiting the one or more ranges is caused by repeatedly changing an imaging geometry of the imaging apparatus.

In embodiments, upon revisiting and reacquiring, a collimation aperture of the imaging apparatus is changed at least for one of the ranges.

In embodiments, the changing of imaging geometry includes moving an examination table on which the surrounding object resides.

In embodiments, the one or more findings and/or scores are providable by one or more trained machine learning model.

In embodiments, the at least one of the said ranges, on a revisit, is smaller than on at least one earlier visit.

In embodiments, the system as per any one of the above mentioned embodiments comprises the, or a, visualizer operable to cause displaying, or selectably so displaying, on the, or a, at least one display device , a graphics display of i) any one or more of various projection frames at different doses and for different ranges, or the slice imagery reconstructable from the projection frames at different doses and for different ranges, ii) data in relation to the respective findings including the IQ score and/or the IC score. The graphics display may support a user interface, such as an interactive user interface, in particular a graphical user interface. Such interface may include selector/toggler function to switch between displaying of reconstructed imagery for different ranges with associated information. Such a feature may prevent information overload on the part of the user.

In embodiments, the imaging apparatus is any one of: a medical CT imager, a C-arm imager.

In embodiments, the first or an earlier such scan is a surview scan along an initial or earlier range is that a prior known to include /run past the region of interest. However, unlike in traditional surview based imaging protocols, where the scan proceeds directly to scan the ROI as localized in surview and scans there only, in the proposed method there is no need for such localization: the whole or large parts of the initial surview range is scanned and rescanned at places until quality is on target for the whole or most of the initial range, which will then also include, at one point, the ROI, as the initial range was chosen upfront to be long enough to cover ROI.

In another aspect there is provided an imaging arrangement including a system as per any one of embodiments, and any one or more of: the imaging apparatus, a display device, a storage on which is storable the projection frames and/or slice imagery reconstructable from the projection frames, the one or more trained machine learning models.

In another aspect there is provided a method of tomographic imaging, comprising:
- receiving a scan request for a region of interest in a surrounding object;
- in at least one scan of an imaging session with a tomographic imaging apparatus, acquiring, along an acquisition axis of the imaging apparatus, multiple projection frames for some ranges along the acquisition direction for the surrounding object, optionally with application of an image quality, IQ, booster operation; and
- whilst the imaging session is ongoing, based on a quality assessment in relation to the acquired projection frames, revisiting one or more times, at least one earlier range and re-acquiring there fresh projection fames with application of the image quality, IQ, booster operation, until the quality assessment is indicative of the quality being on target or until a stopping condition is met.

In embodiments, the method comprises reacquisition of fresh frames at other one or more ranges or same rang(es), with application or re-application of the image quality, IQ, booster operation, if the quality assessment is off target, and to cause the so revisiting and to continue reacquiring other fresh frames there, with respective (e.g., prior to some or each re-acquiring) application of the image quality, IQ, booster operation, until the quality assessment is on target or the stopping condition is met.

In embodiments, the said image quality, IQ, booster operation includes one of i) increasing dose, and acquiring or re-acquiring at a higher dose than before, or ii) combining previously acquired frames (in previous pass(es)) and reconstructing same, or combining previous slice imagery from previous pass(es).

In embodiments, the said combing includes adding up or averaging previous frames, or of reconstructed slice imagery.

In another aspect there is provided a computer program element, which, when being executed by at least one computing system, is adapted to cause the computing system to perform the method as per any one of the above-mentioned embodiments.

In another aspect there is provided at least one computer readable medium having stored thereon the program element, or having stored thereon, parameters of the one or more trained machine learning models.

In another aspect there is provided a use of a scan protocol for a tomographic imaging modality, conducted for a region of interest, in which, during the conducting of the scan protocol, one or more subsidiary finding is being picked up for at least one subsidiary region of interest, other, and in addition to, at least one finding for the region of interest.

As said, the IQ booster operation is configured to improve IQ and hence diagnostic certainty. The IQ booster operation is preferably applied in between scan passes. Ranges to be scanned in a given pass are scanned first, the quality is checked, booster operation applied, and then in the next passe(s), some sub-ranges of ranges in the earlier (preceding) pass are revisited and rescanned, and so forth, until all of the initial range was scanned on target, or until stopping condition is met.

As said, the IQ booster operation doses increasing in different passes, or combining (e.g., averaging) projection frames or reconstructed imagery.

In the following however, the principles will be mainly explained with reference to the dose increase IQ booster, but the over frame or imagery averaging is equally envisaged in the embodiments. Both, dose increasing and averaging, may be done in some embodiments, or only one variant is done, and not the other. As a further option, the at least two IQ booster operation variants (dose increase vs averaging) may be done, each in turn over the passes, in a regular alternating pattern or at random.

The quality scores/parameters as used herein pertain to image quality or the quality (e.g., certainty) of the diagnostic finding.

That the proposed system and method is based on projection frames (instances of projection data for different projection directions per position) on the said acquisition axis (rotation axis) of the tomographic imaging apparatus) includes assessing quality of the projection frames as such, or of the quality of the slice imagery reconstructable therefrom.

The proposed new protocol addresses a deficiency in existing scan protocols in that those existing scan protocols, in particular purely surview based ones, do not take into account the fact that diagnostic certainty can be achieved with very low levels of dose in certain parts of the scan range, if the images of the low dose scan are properly assessed. An example would be one organ (region of interest), that needs to be studied in great detail, whereas surrounding tissues are only screened for large anomalies. The proposed adaptive scan protocol is more dose efficient and less harmful for the patient.

The proposed setup effectively "fuses" surview scan and later diagnostic scan of traditional scan setups into one, single, operational scheme, with stepped or continuous ramp up of dose, until IQ of the FOV with the ROI in it has the requisite level. With this scheme there is no longer a dedicated earlier surview scan. The various scan stages provide together imagery at different doses for different parts of the scan range, and thus it may be possible to pick up incidental findings on the way, on top of providing the diagnostic scan of the actually requested ROI. The output is a collection of tomographic imagery at different IQs that together "tile" the whole range, and provide diagnostic info on ROI and potentially on subsidiary regions as well.

What is proposed herein is the said new protocol, which is of iterative character: it may call for an iterative sequence of scan passes with increasing levels of dose but decreasing scan range. The decreasing range of the second and, if needed, subsequent scan is based on the quality and diagnostic certainty achieved in the first/earlier scan, and so on. In each iteration or pass, quality and certainty are assessed by applying the said machine learning models, either on a per slice basis (which is suited for speed), or on the whole image volume or parts thereof. The sequence continues until diagnostic certainty is achieved over the requested field-of-view which is known a priori to include the region of interest. In other words, the ranges that are scanned along the patient will get "sparser", as the certainty is improved with increasing dose or applicable IQ booster operation.

*"user"* relates to a person, such as medical or clinical personnel or other, operating the imaging apparatus or overseeing the imaging procedure. In other words, the user is in general not the patient.

*"object region"* is used herein in the general sense to include animate *"objects regions"* such as a human or animal patient, or anatomic parts thereof but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient.

*"range"* a length segment of the tomographic rotation axis Z, often parallel to, or coincidental with, imaged patient's longitudinal axis.

### BRIEF DESCRIPTION OF THE DRAWINGS

Exemplary embodiments of the invention will now be described with reference to the following drawings, which, unless stated otherwise, are not to scale, wherein:
Fig. 1 illustrates a block diagram of a medical imaging arrangement as envisaged herein in some embodiments;
Fig. 2 shows a block diagram of a control system for X-ray based imaging as envisaged herein in some embodiments; and
Fig. 3 shows a flow chart of a computing-implemented method for X-ray imaging, in particular a computing-implemented method for controlling X-ray imaging modality, in particular still for controlling a tomographic imaging modality.

### DETAILED DESCRIPTION OF EMBODIMENTS

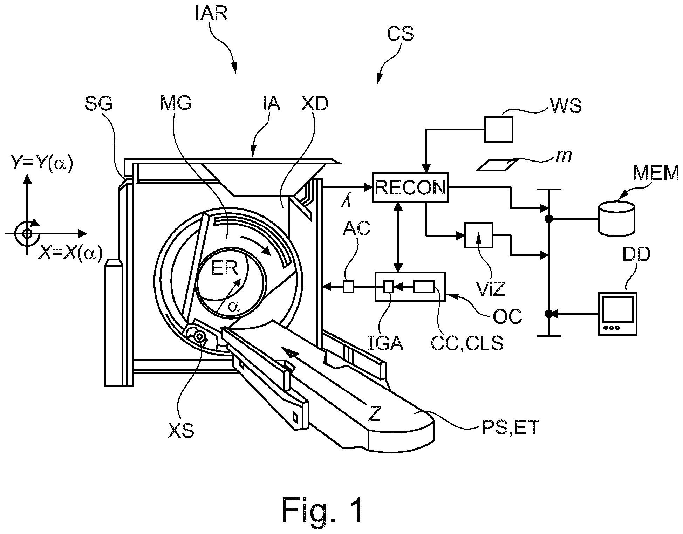

With reference first to the block diagram of Fig. 1, this shows various components of a medical imaging arrangement IAR as envisaged herein in embodiments.

Broadly, the medical imaging arrangement includes an X-ray imaging apparatus IA ("imager") shown to the left of the Figure, and, optionally, an imaging supporting computing system CS coupled thereto. Broadly, the medical imaging apparatus IA is preferably X-ray based, and is operable in an imaging session to acquire measurement data λ of a patient PAT at least partly residing in an examination region ER of the imaging apparatus. The computing system CS may be operable to process the measurement data λ into image data *m.* As will be explained in more detail below, the imaging arrangement may include novel imaging control circuitry CC, in the following referred to herein as (imaging) control system CLS, that is operable to administer a novel imaging protocol. That is, the control system CLS is operable to control the manner in which the said measurement data λ is acquired.

The said measurement data λ may include in particular projection data as acquired from the patient PAT upon exposure of the patient to X-radiation emittable by the imager IA's X-ray source XS. The X-radiation so emitted interacts with patient tissue, and is modified due to the interaction. It is the so modified X-radiation that egresses the patient at the far end, and is then registered as intensities by imager IA's X-ray detector XD. Registered intensity measurements are converted into digital data (that is, into a set of numbers) by data acquisition unit (not shown), and is then made available as the said projection data λ for processing by the computing system CS, or other data consumer. The measurement data, in particular the projection data λ, may be forwarded through wired and/or wireless data link from the X-ray detector XD to the computing system CS, or other such data consumer.

Turning briefly to the computing system CS first, this may be fully or partly integrated into the imaging apparatus. It may be arranged in the examination room, or outside it in the same medical facility, or it may be at least partly remotely located as one or more servers or edge computing modes, depending on the computing/networking architecture as needed. The computing system CS may be implemented as a workstation WS.

Broadly, the computing system CS includes various (sub-)data consumers that process the projection data for medical needs. One such data consumer may be a reconstructor RECON. Specifically, reconstructor RECON may apply a tomographic reconstruction algorithm to compute from the projection data sectional (tomographic) imagery *m,* of main interest herein and obtaining such imagery may form the objective/purpose of the imaging. Reconstruction algorithms can be understood as mapping from projection domain into image domain. The earlier is a data space located at the detector whilst the latter is a data space located in the examination region ER. The reconstruction algorithms proceed by populating voxel grids of the image domain with image values. The reconstruction algorithms may use backprojection operations. The cross-sectional imagery *m* may be volumetric to cover/represent a 3D space portion in which at least a part ROI of the patient PAT is located. The tomographic reconstruction algorithm may include filtered back projection (FBP), algebraic, or iterative reconstruction, or any other. The imagery *m* may be located in a plane(s) perpendicular to a rotation axis Z of the imager IA. The rotation axis, also referred to herein as imager IA's acquisition axis, usually coincides with, or is parallel to, the patient's longitudinal axis during imaging. Thus, the reconstructor RECON may produce such sectional imagery along different ranges along the rotation axis Z. The projection data and/or the volumetric imagery may be forwarded through a data bus or other data link to one or more various other data consumers for processing there. For example, the imagery *m* may be stored in a memory ME volatile or non-volatile, such as in a picture archiving and communication system (PACS), a hospital information system (HIS), or any other.

The imagery *m*, or indeed the projection data on which the said imagery is based, may be processed by a visualizer VIS to generate a graphics display thereof. Image values that make up the imagery *m* may be mapped to gray-or color values of a palette. The mapped image values may then be used to drive video circuitry of a display device DD. Thus, the visualizer VIZ may generate graphics display that represents the acquired imagery for view on the display device DD. This can be then used by a medical user, such as a radiologist, to "read" the imagery visually. In addition, or instead of displaying the imagery, the imagery may be processed using artificial intelligence ("AI") diagnostic aid(s), on which more below but in different context, to derive automatically clues on diagnosis, therapy or any other. In this or other cases, no displaying of the imagery may be needed with such automated image analysis, but may still be done. Whether or not such AI is used, radiologists or other AI may draw up a report alongside the reviewed imagery, and pass this on to the responsible medical professional who has seen the patient, and who may have requested the imaging in the first place. Any other uses of the imagery also envisaged herein may include radiation therapy planning, or any other, as the case may be.

Operation of the imaging apparatus IA as such will now be described first, before turning to the control system CLS for more detailed explanation. Generally, tomographic imager IA may be in particular a diagnostic CT scanner. Thus, imager IA may have a characteristic "doughnut" shaped gantry GT, SG, MG, with the opening around the anulus constituting the examination region ER, through which the rotation axis Z is run, and in which at least part of patient PAT resides during the imaging/data acquisition operation. Imager IA's radiation source XS and/or detector XD may be referred to herein as "acquisition equipment" (of imager IA).

Thus, the imaging apparatus IA is preferably of the tomographic type, and is thus configured for multi-directional projection image acquisition. The imaging apparatus IA is thus capable of acquiring projection images λ along different projection directions α relative to the examination region ER, and thus the anatomical region ("ROI") of interest of the patient. Acquisition can be done in embodiments by a rotational system where at least the X-ray source XS is arranged in a moveable gantry MG. The movable gantry (and with it the X-ray source XS in embodiments) is rotatable in a stationary gantry SG around the examination region ER in which the patient/ROI resides during imaging. Opposite the X-ray source in the movable gantry there is the X-ray detector XD which may rotate with the gantry and X-ray source around the examination region ER to realize the different projection directions α. The CT scanner setup as illustrated in the Fig. 1 is merely according to one embodiment, and other tomographic imaging equipment, such as interventional C-arm or U-arm scanners, cone beam CT arrangements, mammographic imagers, etc., are not excluded herein. C-arm cone beam imagers may be preferred herein in some embodiments. In addition, the multi-directional acquisition capability may not necessarily result from a mechanical rotational system such as shown in Fig. 1. Non-(mechanical) "virtual" rotational imaging systems are also envisaged, such as in CT scanners of the fourth or higher generation, where multiple X-ray sources are arranged around the examination region in a source annulus for example. In addition, or instead, the detector XD may be arranged as a detector annulus around the examination region. Thus, in such systems there is no mechanical rotation of the X-ray source XS or detector XD, or of both, but by switching source/detector in sequence, a virtual rotation is effected, which also allows said multi-directional imaging.

Step-and-shoot, or, preferably, helical scanning protocols may be used herein to cover the applicable range (on which more below) along axis Z, whilst acquiring the projection data λ in rotation of acquisition equipment (detector XD and/or source XS) around the axis Z.

Overall operation of the medical apparatus. such as commencing the data acquisition of projection data λ, or other particulars on how the imaging operation session unfolds may be controlled automatically and/or by manually by a user via an operation console OC suitably coupled by suitably interfacing with the imaging apparatus IA. The operation console OC may be computing device, including data processing infrastructure enabling the computing, such as digital data processing circuitry (e.g., processor (CPU)), memory, I/O interfacing, a bus system interconnecting some or all of the aforementioned infrastructural components, data networking circuitry, etc. I/O interfacing may allow imaging commands/imaging protocol data to be read in, and/or provided by user though suitable input interfacing (keyboard, GUIs, etc.).

The operating console OC may be arranged at the imaging apparatus IA. It may be data-coupled wirelessly and/or in a wired manner with the imaging apparatus IA. The operating console OC may be physically situated in a preferably radiation-shielded examination control center/room, usually, but not necessarily, adjacent to the examination room in which the imaging apparatus IA as such, with the patient PAT, is present.

Measurement control operations/parameters as supported by the operating console OC includes in particular setting the dose *D* of the X-ray source XS, and/or changing the imaging geometry via an image geometry adjuster IGA. In general, imaging geometry includes herein the mutual spatial arrangement or configuration in 3D space between i) the patient, and ii) acquisition equipment (including the X-ray source and/or the detector). The imaging geometry setting may be adjusted mechanically, either manually, automatically or semi-automatically through suitable actuators AC responding to control commands issued by the operator console, either automatically in processing the imaging protocol, or as may be input by the user manually as mentioned above.

Turning now to the control system CLS, this may be fully or partly integrated into the operator console CS. The control system CLS as envisaged herein, and whose operation will be explained in more detail below, is operable to control some or all of the above-mentioned parameters, in particular, dose and/or imaging geometry.

As mentioned above, the control system CLS effects the said new imaging protocol, preferably, an X-ray based tomographic imaging protocol, in order to achieve better efficiency ratio(s). That is, the proposed control system CLS allows extracting more information for every dose unit expended. In particular still, the newly proposed control system CLS is no longer reliant on the previous dichotomy between prior surview scans, and subsequent diagnostic scan once the location of the ROI is found.

In departure from such previous surview based scan protocols, the proposed new protocol is more flexible and administered in a unitary manner. To this end, the control system CLS causes an IQ booster operation to be applied in the protocol. One such type of IQ booster operation contemplated herein amongst others, and indeed preferred herein, includes increasing (X-radiation) dose that is expended over time and space along axis Z to cover a preset initial range (a spatial section/segment along axis Z) that is a priori known to include the regions of interest, ROI. Imaging ROI is the (primary) purpose of the imaging. In the new protocol, dose is ramped up continuously, or in steps, starting from a very low dose, up to a target dose that is needed for the region of interest (the purpose of the imaging). The dose is expended spatially over the range Z, with revisits (rescans) of certain ranges along Z in cycles, also referred to herein as "passes". This spatial and temporal dispensing of dose along axis Z is geared so as to increase the odds of discovery of even incidental findings along the way as the dose is ramped up over the scanned range along the axis Z. This affords better, more efficient, imaging with better image-based intelligence extractable for a given dose unit spent. The manner in which the dose is expended in magnitude and in space (along axis Z) is controlled by an IQ based quality check, as will be explored in more detail below. Whilst it remains so that that it is the ROI that is ultimately imaged at the requisite dose, some dose is spread out over the surrounding ranges and is not, a priori, restricted only to the range along axis Z where the ROI is located. It is preferably the whole of the initial range *r0,R*, which is known to include the ROI purely on basic medical-anatomical grounds, that is scanned until certain image quality ("IQ") target(s) are met preferably, over the whole initial range. This increases the odds of detecting incidental findings for other regions ROI', as such different from the actual region of interest ROI. This allows better imaging efficiency (ratio) at about the same overall dose costs as was previously spent on traditional surview scan-based protocols.

Another type of IQ booster operation is averaging over frames. Thus, in this embodiment, dose may remain the same in the next scan, but the two or more images or projections frames from current scan *i* and previous scan *i-1* are averaged or otherwise computationally combined, to thereby reduce noise, potentially yielding higher q's and/or p's this way. However, in the following, main reference will be made to the IQ booster operation in term of dose increase. Whilst this is preferred herein, the other options including the over frame averaging or combining is also envisaged herein, and the below is, mutatis mutandis, of equal application to IQ booster operation via combining /averaging projection data frames λ.

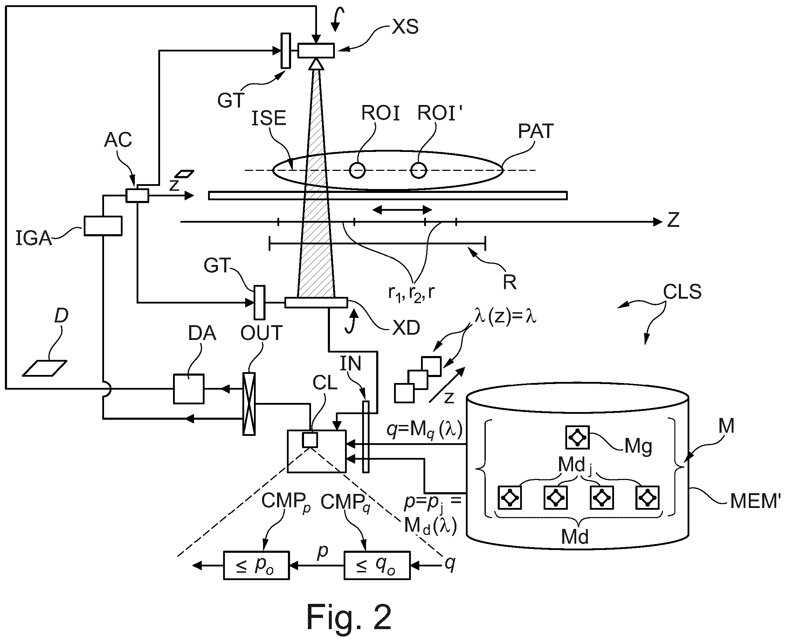

Operation of the proposed control system CLS is now described in more detail, with reference to the block diagram in Fig. 2.

Patient PAT may reside in the examination region on a patient support PS, such as an examination table ET. Patient PAT has his or her longitudinal axis aligned at iso-center ISE with the rotation axis Z of the tomographic imaging apparatus, such as a CT scanner of Fig. 1 or other.

For present purposes, patient PAT's body may be considered a surrounding in which the region of interest ROI (which is the purpose of the requested imaging) is located and needs examining. For example, structural/anatomical details or features of the region of interest ROI may be needed in order to inform diagnosis and/or therapy. But there may also be one or more of the said other, "subsidiary" regions ROI', not of interest as yet, but which nevertheless may exhibit some pathology, and such can be picked up "along the way", as incidental findings in the newly proposed imaging protocol as administered by the proposed X-ray imaging control system CLS.

Broadly, and in yet more detail, some or all functionalities of the control system CLS may be performed by one or more computational entities/nodes, etc. They may be proximately located to the imaging apparatus following edge computing principles, but some or all of the computational nodes may be located remotely, on one or more servers or other nodes, and are accessible through a data communication network. As said, in some embodiments the control system CLS is partly or fully integrated into the imager IA, such as may be integrated into imager IA's operating console OC or elsewhere, such as in the supporting computing system CS.

Broadly, the control system CLS is operable to receive the projection data *λ* as acquired in frames along the rotation axis Z. As will be explained in more details below, one or more ranges *r, r0, rj* along the axis Z are covered to acquire multiple sets of projection frames for the respective range, along the axis Z. One or more tomographic/cross-sectional images *m(z)* may be reconstructable by operation of the reconstructor RECON from the frames, with each such reconstructed slice or volume image m(z) pertaining to given position or segment on the rotation axis z *∈* Z. If the m is a volume, z may pertain ot a reference point, such as one of its ends, or center, etc.

The control system CLS processes the received projection data to compute a control signal. In more detail, the proposed control system CLS processes input data received at one or more of its input ports IN, and processes the same to produce the output data. The output data includes control signal(s) provided at one or more output ports OUT to so administer the new imaging protocol. The control signals may be used to adjust dose and/or imaging geometry in the ongoing data acquisition/imaging session, or more generally control, cause, adjust etc., the IQ booster operation.

The input data is based on some projection data λ. For example, the input data may include the projection data λ is such. Preferably however it is the reconstructed imagery *m*(z,λ) reconstructable from the acquired projection data λ that is processed by the control system CLS. Thus, the control system via its interface IN may be interfaced with the reconstructing unit RECON.

The input data further comprises certain quality scores *s=*(*p,q*) applicable to the image input data, such as to the projection data or, preferably, to the reconstructed imagery *m.* Thus, in preferred embodiments, the input data is a dual data stream that comprises i) the image data (preferably reconstructed imagery *m*(z,λ) and ii) the quality scores (*p,q*) of the image data at i), to so produce at output port OUT the output data. The output data is configured to be received at suitable control interface(s) of circuitry of the imager IA to execute and influence the acquisition of the projection data in the current imaging session. Thus, based on the output data, which is itself at least partially based on already acquired projection data, it is the manner (e.g., location and/or dose) of acquisition of newly acquired projection data that is controlled by the earlier projection data. Thus, the system may be understood as an iterative protocol.

The output data/signals produced by the control system CLS may be configured to cause adjusting the imaging geometry of the imaging apparatus. The control system CLS interfaces with the image geometry adjuster IGA and so passes the output data/signals to the image geometry adjuster IGA. The image geometry adjuster IGA interprets the control data/signals. Based on the interpreting, the image geometry adjuster IGA in turn sends specific signals to one or more actuators AC to so effect the desired imaging geometry adjustment. For example, in some embodiments, it is in particular the relative spatial arrangement of the patient versus the X-ray source XR and/or detector XD that is adjusted. A given imaging geometry defines the field of view (FOV) and adjusting the imaging geometry in general causes a change in FOV. More particularly still, there may be a relative translation movement effected between the X-ray source/X-ray detector, and the patient. In some, if not most cases, this can be done by advancing parallel to the rotational axis, indeed along the axis, the examination table ET, PS on which the patient PAT resides (in general, lies) during the imaging session, to so allow acquiring different sets of projection frames along different segments, referred to as ranges *R, rj* along the *Z* axis and thus along patient's longitudinal axis. The term "projection frames" refers essentially to projection image data and may be understood as data obtained per detector readout and can be associated with a particular imaging plane intersecting the rotation axis *Z* at a point *z,* in which plane the respective reconstructed image m(z) can be thought to be located. Thus, by extension, the projection frames may be associated with the respect point *z* on axis *Z.* In general, plural projection frames λ(z) are acquired along different directions α for given position z on the rotation axis Z. The respective plurality of projection frames *λ(z*) are then reconstructed into the respective tomographic (cross sectional) image *m(z)* for the said position z. Thus, the respective image *m(z)* can likewise be said to be associated with the point *z* on axis Z. Each such image *m(z)* represents a cross-sectional view across the patient in a section plane that intersects axis Z at z. The intersection point may be understood as the mentioned reference point for a (small) volume segment, as the reconstructed imagery *m(z)* has a certain thickness in *Z* direction so can be conceptualized as a slab of voxels, rather than a geometrical planar set of such voxels. However, in approximation it may be admissible to conceptualize imagery *m(z)* as planar as their thickness is small (for a given single slice) in comparison to the length of the ranges scanned.

In addition to so changing the imaging geometry in a particular pattern as will be explained in more detail below, it is also dosage *D* of X-radiation emitted by the X-ray source XS that is adjusted by the control system CLS via its dosage adjuster DA. The dosage adjuster DA admitters the IQ boost operation. The adjuster DA includes interfacing circuitry which allows interfacing with circuitry of the X-ray source XS. Based on the control data *D* output by control system CLS, the dosage adjuster DA may cause adjusting operating amperage and/or voltage of the X-ray source. The X-ray source may be an X-ray tube. In such X-ray tube systems, the energy (and hence dose) of the emitted X-ray radiation can be adjusted by adjusting the cathode current and/or accelerator voltage between cathode and anode disc.

In general, the dose adjustment data *D* as produced by the control system is to cause a non-uniform dose profile *D(z)* along the scan axis *Z,* based on the quality scoring s, as will be explained more fully below. Specifically, it is envisaged herein to ramp up, continually or in discrete steps, the X-ray source from a very low initial dose comparable to that previously used for a surview scan (but may even be lower), to a medically prescribed maximal dose needed for the patient based on their bio-characteristics, such as BMI, etc., and the actual region of interest ROI, such as the liver or lung that is to be imaged primarily. Once ramped-up to a or next level, different ranges *rj* -- two such rages *r1,r2* are shown in Fig. 2, purely for illustration but there may be more or less than two -- are scanned, commencing from an initial, all encompassing, range *R* which may amount to a whole body scan along the whole of the patient's longitudinal axis. However, such whole-body scan may not necessarily be needed, and the initial scan range *R* is shorter. Thus, the initial scan range *R* may be chosen just long enough to scan an anatomical segment of interest along *Z* of the patient's body, such as in an abdominal scan, where the range R may run from the patient's hip to the neck, or in a leg scan where the initial range R may run from the hips down to the patient's feet. Similar for head imaging, where the applicable anatomic segment to be covered by the initial range R runs from patient's neck to the top of the skull, etc. The initial range is chosen long enough to ensure the actual region of interest ROI is covered.

Based on the said quality check of the mentioned quality scores, to be described more fully below, the different one or more (sub-)ranges *r1, r2* of the initial range *R* are re-scanned, but now with a different setting of the IQ booster operation, such as at a different dose than the one used for the earlier range, such as the initial range R. The one or more subranges are not necessarily overlapping, but may be instead discretely arranged at gaps along axis Z. In subsequent one or more passes or "cycles", new sub-sub-ranges are re-scanned at ever higher dose, with (sub-sub-... -sub-)ranges in general becoming smaller compared to a one or more previous ranges previously scanned at a given location z as the imaging session controlled by the newly proposed protocol unfolds. At the same time, the dose is ramped-up and subranges *rj* are then re-scanned at higher dose, in later cycles/passes. The spatial distribution and respective, in generally decreasing, size of the sub-ranges *rj* over the scan cycles/passes will depend on the respective scores each range attracts, and the quality check based on such scores. In the proposed new protocol, progressively smaller ranges are rescanned in iterative cycles/passes at progressively higher dose.

The manner in which the protocol evolves, that is, which sub-range(s) are to be re-scanned at pass j at progressively higher dose is a dynamic process, and is not, as such, preset at the outset. Instead, it is determined by the quality score for the imagery *m(z,j-1)* reconstructed from projection data acquired in earlier passes, with index *j* indicating the respective cycle/pass. Scan position z /subrange *rj* including this position z. may or may not need revisiting/rescanning, depending on the score of *m(z,j-1).* Rescanning in this manner causes the mentioned non-uniform dose profiles *D '(z)* over axis Z to be used, as some ranges/positions are no longer rescanned whilst others are, at higher dose than before. In between passes, when adjusting the imaging geometry for the next subrange *rj* scan, radiation exposure may be temporality disabled by collimation, by grid switching, etc., or may be left on, as needed. Exposure is re-enabled once the imaging geometry is adjusted with the next subrange(s) in the field-of-view (FOV) in the revisit, and so forth. During administration of the protocol, that is, during the one or more various passes of rescans for ranges *rj* at different doses *Dj,* the patient remains in the examination room, on the patient support PS, and at least partly in the examination region ER of the imager IA.

Thus, in the proposed control system CLS, various (sub)ranges *rj* along axis Z are re-visited and are re-scanned if needed at progressively higher dose at each visit, compared to an earlier visit there. This is done until the scanned ranges, including at least one r1 for the actual region of interest ROI, have received sufficient dose as can be measured by the mentioned quality scores *q,p,* which will be discussed below shortly. As will be understood from the above-described manner in which the protocol is set up, as the imaging protocol unfolds, the other, subsidiary, regions ROI' are scanned at least at some ranges at some point in time, with sufficient dose, and the proposed system allows thus potentially picking up subsidiary findings in relation to such subsidiary region(s) ROI'. Although such findings may not have been of interest as such initially when the imaging for called for by clinical user in relation to the actual region of interest ROI, such picking up of incidental findings may still be beneficial for patient's health, and for public health care management more generally. Thus, the efficiency score can be increased: whilst at the time when the imaging was called for there may have been a pressing need for examining the specific region of interest ROI as such, healthcare can be run more efficiently if findings in relation to other regions ROI' are picked "on the fly" and such subsidiary regions ROI's. They may be able to receive therapeutic attention even before symptoms manifest. The sufficiency of dose for the subsidiary region ROIs) is in general different from (in generally less than) the dose deemed sufficient for the ROI, and is measured differently based on the scores as will become apparent below. Thus, the proposed system allows a more holistic diagnostic information gathering, thereby increasing the efficiency ratio.

Indeed, some of the benefit in the proposed imaging protocol rests on the observation that some diagnostic machine learning models M can cope with surprisingly high noise levels/low SNRs environments: it has been found that they may be thus able to pick up findings in relation to subsidiary regions at much lower dose and under high noise than the target dose that may be needed for actual region of interest ROI. Thus, instead of expending low dose merely and solely for navigation purposes as done before in low dose surview imaging protocols, the newly proposed protocol is configured to make diagnostics use even of low dose, up towards the target dose that is prescribed for the actual ROI.

As mentioned above at places, whether or not to re-scan at given location/ subrange, and what dose to use there, is a function of the quality assessment data (*p,q*)*,* also referred to above as the quality scores s. Such scores *s=(p,q)* may be provided preferably, but not necessarily, by one or more machine learning models M previously trained on training data. Statistical models may also be used, instead or in addition. One such model, referred to as the image quality assessor model M_{q}, may have been trained based on projection data or reconstructed imagery in order to assess whether basic image quality requirements, such as noise level and/or motion artifacts, are met. Thus, the model M_{q} may provide upon input of a given reconstructed image *m,* an associated image quality level score *q=q(m).*

Another category of machine learning models, referred to herein as diagnostic model(s) M_{d ,} as may be used herein, is configured to operate on the diagnostic level. That is, such model(s) M_{d} may provide, for a given reconstructed imagery *m* for a given location z, and having a sufficient image quality level *q,* a diagnostic assessment *p.* Thus, data *p* may pertain to a specific sub-region of the image that may be flagged-up as a suspicious image structure. The diagnostic data *p* may further include in addition, or instead, a proposed diagnostic finding, such as whether or not a pathology, disease or condition may be present as per a given image m reconstructed during the unfolding of the imaging protocol. There may be a bank of such diagnostic models M_{d}={M_{dj}} each model M_{dj} trained to detect a particular single or related group, of pathology j. Models M ={Mq, {Mdj} }may be held in one or more memories MEM', and can be accessed by control system CLS for quality assessment service. The collection of models may or may not be part of system CLS.

Thus, the bank of ML models may provide, for the given image *m,* a set of findings *pj,* each such finding relating to a respective pathology j, possibly known a priori, on general medical knowledge grounds, to represent likely comorbidities in relation to the ROI. For example, heart problems may cause vessel damage in parts of the vessel system located away from the heart. Alternatively, single or fewer models than the number of pathologies envisaged may be used that can supply diagnostic scores in relation to more than one pathology. Thus, this bank Md = {Mdj} of one or more diagnostic models can be chosen up-front to have diagnostic capabilities that pertain to generally known comorbidities that are clinically known to be often associated with a primary finding sought for the region of interest ROI. Thus, the bank of diagnostic models may not only include a model to be able to detect a finding in relation to the region of interest ROI, but may also include one or more other models that are able to detect other findings in relation to the same region of interest, and/or, more importantly, in relation one or more subsidiary findings for other one or more subsidiary regions ROI' that that may well be located elsewhere from where the region of interest ROI is located, along, parallel or perpendicular to, the imaging rotation axis Z. Preferably, the one or more diagnostic finding score *p=pj* may include, or represent as such, not only the finding as such, but also a certainty score which pertains to the diagnostic finding. In this connection, a higher certainty may be equated with a better quality score *p.* For example, the score *p* may be "*j-*>*95%*", which means that the model attaches a *95%* certainty that finding j is valid, such as may be provided by classifier type models, mainly envisaged herein. In some such classifier models the finding pj may be localized in the input image *m* itself, such as by a bounding box or other mark-up facility to so indicate the underlying image structure that led to model conclude for the finding it did. Such an example 95% certainty may be considered herein a better, more desirable score than, say, an 80% certainty finding. In the following, the designation *"pj"* or *"p"* for the diagnostic score may be used to refer in particular to this certainty element in relation to the diagnostic finding. Also in the following, for simplicity, reference herein will be made to singular "the" diagnostic model M_{d} and the certainty score *p,pj* in relation to its finding, it being understood "the model" may be a bank (a plurality) of such models *M_{dj}.* Thus, the reference "diagnostic model M_{d}" may be understood as shorthand for a single such model, a generic reference to such a model, or as a reference to plural models collectively, such as the said bank of models, if any.

The certainty score attached to a certain finding j may be a statistical score or Bayesian degree of "belief', such the above-mentioned p% percentage value returned by classifier model Mp for a given class j representing a certain condition, malady, or other clinical result *j.* The certainty score may be provided natively by the trained model in a separate channel such, or may be inherently part of the native output as in the above-mentioned classifier type models. Other ML model types, such as regressors (also envisaged herein in some embodiments for model Mp) not natively providing such a certainty result, may be analyzed by gradient-based methods, or others, to ascertain such a certainty value in an analytical post-processing step. For example, it may be examined how the parameters of the model change with respect to internal parameters and/or with respect to images values of the input image *m* applied to the model Mp, and this can be compared against thresholds. Or it is examined how the output changes with input or internal parameter, etc. Numerical instability, or large changes/gradients may indicate a lower certainty, as compared to higher certainty, where no such larger changes are ascertainable. Numerical methods such as gradient based methods can be used for this. The exact functioning of the certainty scheme for ML models trained on training data as such has been described elsewhere, such as by *M Abdar et al* in their review paper "A review of uncertainty quantification in deep learning: Techniques, applications and challenges", published in Information Fusion (Elsevier), vol76, pp 243-297 (2021).

The above described scores *s=(p,q)* may be combined or fused or otherwise analyzed collectively to derive the control signal. For example, in embodiments, the control system CLS may include a control logic/circuitry CL (also referred to herein as a control module), which is operable to perform a comparison operation based on the scores s, in order to derive the control signal. As mentioned before, the control signal(s) may cause an imaging geometry adjustment, to so effect the revisit of the range(s) *rj* to be rescanned, with dose adjusted for the re-scan, or control IT booster operation otherwise. More particularly, a two-stage sequential control arrangement may be administered by the control logic CL, as indicated schematically in the lower left of Fig. 2. The first stage may be formed by the image quality model M_{q} whilst the second is formed by the one or more diagnostic model M_{d}. The respective model processes a given image *m(z)* to yield scores *p=p(m), q=q(m)* for that said image *m* reconstructed from projection data λ*(z*) collected at a given location z (we may on occasion drop the *"z"* index to disencumber notation). Respective comparator CMPⱼ may be used, one CMPp for the diagnostic data *p(m(z))* supplied by model Md when applied to image *m(z),* and the (at least one) other comparator CMPq other for the quality data *q(m(z))* supplied by quality model Mq when applied to same image *m(z).* In particular, it is first the quality data q that is analyzed by comparator CMP_{q} and, based on this comparison, data flow is passed conditionally onto the next one or more comparator CMPp that then compares the diagnostic data p for certainty quality. A respective reference threshold *p0* at diagnostic data comparator CMPₚ and *q0* at IQ comparator CMP_{q} may be used. More particularly, the processing may be conditional on the outcome of the comparison. If a comparison at either comparator satisfies the thresholding, the current image *m* is said to be in target, either in terms of quality *q,* or in terms of certainty of findings *p.* This, if given image m yields a quality *q>=q0,* it is on target in respect of quality. If *m* yields a certainty of a finding *p >p0,* it is on target in respect of certainty. Note, there is herein, by definition, always a finding with a certainty. For example, if model Mₚ is geared to detect condition *j,* and it is concluded given input image *m* that no such condition is present, this to is also a finding at a certain certainty. For example, model Mp may predict no such condition *j* is present at 95% certainty, thus *p= pj = p(j) = 95%.* Thus, in operation, imagery *m(z)* reconstructed at position z in a given range *rj* is passed into model stage Md,Mp to predict the scores *p,q.* The scores are processed by comparator stages CMPₖ(*k =p,q*) to derive the output control signal which determines in turn whether the region *rj* needs to be rescanned at a higher dose than before, and so o,n until the whole range has been scanned on target in terms of p and/or q, or else if another stopping condition is satisfied that halts the scan protocol. If a bank of model Md are used, the respective certainties values *pj* may be combined such as by averaging, weighted averaging or in any other way to so compute a master certainty score (symbolically denoted herein as "*Σpj*"), and this is then used herein as described above and hereinunder.

Specifically, and with continued reference to the two-stage comparator setup, only when a certain image quality level as measurable against the quality threshold *q₀* is achieved, only then is the respective image data *m* (or its underlying projection data *λ)* passed on for diagnostic assessment by the one or more diagnostic models M_{d} in the next stage to find *p.* Thus, for those images for which IQ as per score *q* is off target, there is no finding and related certainty *p* computed at all for such images, at least at that point in time. Only when, after a repeat scan by revisiting the same location *z* of the off-target image *m(z),* a newly reconstructed version *m '(z),* based on higher dose projection data is obtained, and *m '(z)* is then found to be on target in relation to IQ as per new score *q,* may such image *m '* then be passed on to the next stage for computing finding at certainty *p.* It is then the new score *p(m')* that is compared at the next stage CMPp, and so forth for each reconstructed image *m(z)* along axis *Z.*

More particularly, the progressively smaller ranges *rj* re-visited in subsequent scans during the proposed imaging protocol are based on such parameters *p,q* as analyzed by comparator stage of CMPₖ of logic CL. For example, once a sufficient IQ has been established by CMPq for a given image *m(z),* the respective range around this z position of image *m* does no longer need to be re-scanned. It is only range(s) rj ∋ *z* that yield off IQ target imagery m(z) (that is, imagery that failed this IQ test as administered by first stage comparator CMPq), that need to be re-scanned, but at a higher dose as before. Thus, the higher dose in the next rescan is set by the control system based on the IQ quality parameter/score *q.* In addition, only those input image data m for a given range that have sufficient image quality are actually passed on for second stage diagnostic assessment by the diagnostic model Md,. And it is the then associated certainty *p* that is compared against the certainty threshold *po* at the second stage. If the certainty *p* is sufficient (is on target), the respective range at *z* will no longer need to be re-scanned, thus no re-visits and associated image geometry adjustments are necessary in the proposed protocol. If, however, the image quality *q* is sufficient, but the certainty score *p* is not sufficient, then the respective range *rj* ∋ *z* may still need to be re-visited, with increased dose exposure.

The dose may be increased in pre-defined steps. Those steps may be equidistant and may be pre-defined. For example, the dose range from the lowest possible dose, such as is used for surview scan, and the minimum dose needed as prescribed by the protocol for the region of interest, may be divided into a pre-defined number of dose levels/steps, each administered based on the outcome of the quality scores/parameters *p,q,* and increased from one level/step to the next which each revisit. For example, tube XS's current may be increased in mA steps. For example, for a chest exam, previously a surview could be done at 1mA for a normal sized patient. A diagnostic scan was then done at a max of 200mA. For each such protocol (chest, abdomen, head), the present dose ramp up may thus be defined as a list of tube currents between the two extremes, which is then iteratively applied in the current setup. E.g., the system FS may apply, in order, starting from 1mA (or less), and then proceed in steps of 5mA, 20mA, 50mA, 100mA, until it maxes out at 200mA (or more). Although two such levels of dose may be sufficient in some simpler cases, preferably more than two such dose levels for ramp up are used, such as three, four, five or six, as in the example, or yet more, as needed and appropriate for the case at hand.

Preferably, the proposed system FS can proceed per slice position z along axis Z when analyzing the imagery for the quality parameters (*p,q).* Thus, scores *s*= *(p,q)* are computed for each image slice *m(z)* at a given location *z* on its own. However, to save time, sub-volumes of slices may be consolidated, and it is such volumes Σ*_{z} m*(*z*) that are then processed as described herein, such as checked for quality *(p, q)* . Thus, in some embodiments, some quality data (p,q) is produced individually for slices m(z) at points z on the imaging axis *Z,* whilst others are processed volume-wise. In embodiments, a mix of point and volume-wise processing envisaged herein, one applied at places, the other at other places along Z. Alternatively, the proposed method proceeds pointwise or volume-wise one at the exclusion of the other, as needed.

Once a certain region *rj* is re-visited because of a lack of image quality or diagnostic certainty or both, the range *rj* is in generally made smaller as in the prior scan(s) by adjusting the collimator (not shown) at the X-ray source, or in any other way. The amount by which the range is decreased in length at re-visits may be pre-set or may be adjusted dynamically based on anatomic knowledge as needed. For example, if it is about the subsidiary finding outside the region of interest, the length of range can be made markedly smaller as compared to re-visits for ranges that are known to include the region of interest, where a more cautious approach is taken, and the range reduction is smaller there. Whilst the exact location of the ROI is not known, as no dedicated surview scan is done and analyzed for location, such a localizer step is no longer needed because of the, in general, convergent nature of the proposed iterative protocol. Thus, it is ensured that eventually the whole range is scanned on target re *p* and *q.* In particular, one may choose length along *Z* of first range *R,* long enough to include ROI with certainty. The length of initial range *R* can be chosen based on medical knowledge. E.g., an abdomen scan may be done to ensure that the ROI, such as liver, is covered. If in doubt, a full body scan may always be done. Thus, in an extreme case, the first range *R* could be full body. By user choosing, via a user interface, the intended "exam type ", and thus the applicable model bank {Mdj} or models thereof, one will implicitly control the shrinking behavior. The "resolution" (the graduation of the spatial steps) by which the range(s) is made smaller, can be set by the user uniformly over the initial length (most practical), or adapted based on the findings of the models, as indicated in the mentioned cautious approach.

Thus, with the newly proposed imaging protocol, in use, the acquisition equipment, in particular the X-ray source and/or X-ray detector, may be seen to scan the patient's longitudinal axis to and from in one or more passes. In this to and from, the system can pick up the subsidiary findings along the way, in re-scanning sub-ranges, possibly with gaps in between with different dose profiles applied at the said ranges, the dose generally increasing on re-visits. Thus, for example, the table ET, PS may be caused to jog to and from in negative and in positive direction along Z, in whichever order best suited to reach the next range, repeatedly, preferably until sufficient certainty *p* and quality *q* is achieved, and no more re-scanning is required.

In the first pass, when the initial range R is scanned initially, a uniform dose protocol may be applied across the overall range, or this can be broadly modulated based on expected in tissue path length through patient. The rescanned ranges *rj* are those that include the *z* positions of those image *m(z)* whose quality, either *p* or *q,* or both is off target, that is, violates the thresholds as operated at comparator stage(s) CMPₖ. Thus, given the one or more z positions of off target *m(z)*'s, a respective margin can be placed to straddle the z's, thus defining the ranges rj to be rescanned next. Because the z's may be spaced apart, the rescannable subregion *rj* may include gaps, so may not necessarily be topologically connected. However, if the margins around the *z* positions are sufficiently close to each other, they may be fused into a single contiguous range, or into one with fewer gaps to make the imaging geometry adjustment simpler.

In the second, or further subsequent pass(es), some ranges may be skipped whilst others are re-visited and re-scanned at a higher dose there. The same higher dose may be applied across all or some of the ranges that are re-scanned. Eventually, in later, subsequent passes, fewer and fewer of the ranges RJ are re-visited at ever smaller widths at the respective locations z that had yielded off target imagery. As the passes unfold, a higher dose is applied at the ever smaller ranges *rj* for each pass *j*, and so forth until substantially the whole region R has been scanned at satisfactory quality and AI certainty, or until a stopping condition is met.

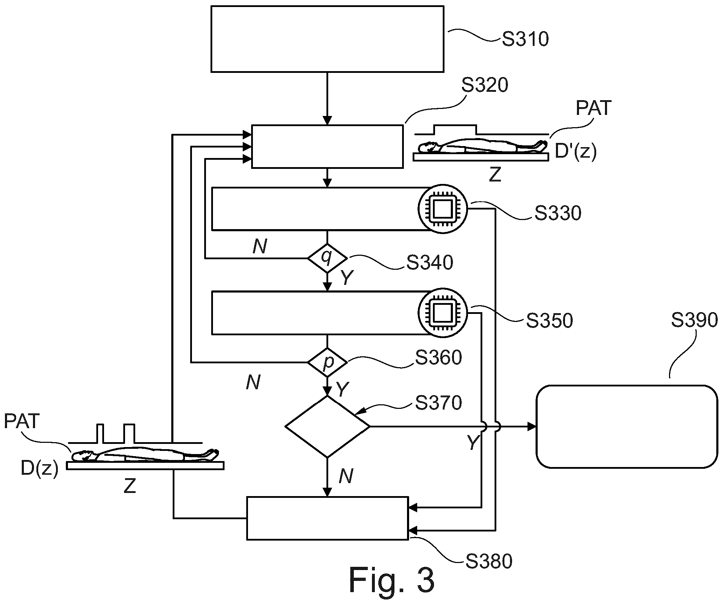

Reference is now made to the flow chart of Fig. 3 which illustrates steps that may be used to implement the above-described control system CLS, but the below described steps may also be understood as a teaching in its own right.

Initially, some remarks on terminology may be due: in this disclosure, below and above, the term "scan" or "scanning", or cognates thereof, relate to the operation of (re-) acquiring some projection data for a position, or along a length, segment, range, etc., along axis Z, and preferably reconstructing topographic imagery *m(z)* based on the projection data acquired for the position(s)/ range covered. Thus, a scan as used herein yields image domain imagery *m(z).* A "rescan" or "revisit", or cognates thereof, relates to the same operation, except that fresh projection data *λ'*, possibly for the same position or range, is re-acquired, but at a higher dose and new imagery *m '(z)* is reconstructed. This new imagery *m'* may be understood as a higher IQ version of the earlier image *m(z),* on account of the higher dose being used, thus improving, if needed, SNR. Thus, the described method (and system describe above) operates mainly on image domain data *m,* and the below and above is mainly confined to such image domain embodiments, cognizant of the proviso that the same operations in the following steps can be done instead entirely or partly on projection domain data λ, and such may be also envisaged herein in such embodiments. In such projection domain focused embodiments, the "scan" operation may thus exclude reconstruction during the unfolding of the image protocol, and the reconstruction may be done as a final post-processing step once the final on target projection data (as measured by *p,q*) are acquired. Thus, in this embodiment, the score s, the *p*'s and q's are taken in projection domain. But, as said, the below will focus on image domain embodiments.

At initial step S310, the initial scan is set up, in particular the first overall range R in the first pass scan, which may be followed up by one or more flow up passes (second, third, etc.) as will be described below. The first, initial, range *R* may be a full body scan, approximately covering the full length of patient's longitudinal axis which axis is assumed herein to substantially be aligned by the tomographic imager's rotation/imaging axis *Z.* However, as a matter of more usual clinical practice, the first range *R* may well be less than the patient's full axis. A whole-body scan is not required herein, provide that the initial range R is chosen long enough that the ROI will fall into imager's FOV. A scan along the relevant for anatomic section, such abdominal, leg, head, etc., may be sufficient. The dosage for this initial scan over *R* may be an ultra-low dose that may be comparable, or less, indeed way less (such as *1-2mA*, or even less), than the dosage usually used currently for surview based protocols. This first dosage may be defined as a dosage profile *D'(z)* over axis *Z.* This may be unform of may take expected in-tissue lengths of patient into account, such as based on patient's physique, BMI, age, etc. A user interface may be used for step S310. The term "revisit" or cognates may be used herein which pertains to imaging geometry adjustments to cause the segments, position, subrange etc., at which rescan is needed, to be within the FOV. This may include moving repeatedly, in a sequence of passes, the patient support PS/table ET on which patient resides during the time the protocol is worked.

In the said step S310, the initial scan range *R* may be defined along the patient's longitudinal axis. This can be done automatically by a camera-based system, or manually by a user indicating the beginning and end of the range in a GUI setup. The number of slices to be reconstructed within the range is defined, e.g., by dividing the length of the range by the desired slice thickness.

At step S320, the first scan is done with the first dosage profile D'(z) over the imaging axis *Z* covering initial range *R,* and hence the ROI but also other region(s), referred to herein as subsidiary one or more regions ROI'. Slices *m(z)* are reconstructed quasi-continuously as the acquired raw data λ permits.

This scan is then analyzed point wisely per section, per slice m(z) along Z or for groups or sub-volumes, as will now be described:
At step S330, using IQ trained machine learning model *Mq,* a score *q* for the image quality of the given images *m(z)* for the initial scan is computed. This IQ score *q* is configured to respond or map out IQ of the *m(z)'s,* taking into account noise level and/or motion artifacts, or any other disturbances or impediments.

At step S340 it is checked whether this computed quality score *q* is on-target, such as is compliant with a pre-defined quality policy. Such policy may be implemented for example by thresholding the *q*'s against one or more threshold reference scores *q0.* If the quality *q* of the currently analyzed image *m(z)* is off target, i.e., is not on-target re *q0,* the respective location or range z is flagged up for re-scan. This rescan can be done immediately once the quality check at S340 concludes for the given image, and possibly whilst imagery from other parts of the range are being reconstructed and processed at steps S330 and S340, or can be deferred until the current pass concludes with all *q*'s gathered for the range that was scanned in the current pass.

As said, some or each slice m(z) is fed into IQ model Mq to check whether basic IQ requirements are met, e.g., noise level and motion artifacts. Preferably this processing is done on 2D slices and can be done very efficiently in parallel as the reconstructed slices become available, and as such does not impede the overall workflow. The output q of IQ model Mq is a quality score per slice. Only slices with a quality score greater than the reference threshold *q0* can proceed to the next stage processing by diagnostic model Md.

Thus, only if a given sectional image or volume image m(z), simply referred to herein as image data m(z), is found to be on-target with respect to quality q0, it is analyzed by another one or more trained diagnostic machine learning model M*d* in the next stage of process flow at step S350.

At this step S350, imagery *m(z)* is analyzed for clinical finding(s), e.g., diagnostic findings, etc. Such findings may or may not pertain to the region of interest, depending on whether it happens to be in the FOV of the imager when scanning for the current position or segment of the current range/subrange (for second pass or later). Eventually, the ROI will fall into the FOV and will be analyzed as above at steps S330-S350 as the imager scans along axis Z to cover the current range. But in its way to or past the ROI, any other region(s), such the said subsidiary, regions ROT', that may be located elsewhere from the region of interest, are likewise ML analyzed for IQ, and, if IQ score *q* is sufficient, for a possible subsidiary finding p. Thus, in general, the bank of models {Mdj} may include models pertinent to the ROI, but may also include additional models that pertain to other aspects of ROI, and, preferably, to subsidiary regions ROI's other and away from ROI, although it was the region of interest ROI that was the purpose of calling for the imaging in the first place. In particular, the scores *p=pj* computed by models for predicting findings are associated with, or represent, respective certainty values that code for the certainty the model attached in coming up with the finding given the input image data *m.*

At step S360, if, for the given image data *m(z)* at a given range *rj, R,* or location/point z it is found that the certainty *p* is not on-target, again a re-scan at the respective location or range is flagged up and/or requested, and can be done immediately whilst other sections are analyzed for its respective certainty or are still being processed at first stage S330 for IQ, or ca be deferred until current scan pass concludes.

In either case, and referring back to checker steps S340, S360, before the rescan, the mentioned IQ booster operation is applied, on which more below.

Some or all slices with a certainty score *q* lower than the given threshold q0 (indicating that the current scan will not be able to answer the clinical question) need to undergo a partial repeat scan. A dose modulation profile over the scan range is calculated which spares the locations of all slices with a high certainty score in the previous step and increases the dose for all other locations compared to the previous scan. Thus, only qualifying slices, with acceptable image quality score q, are fed into a set of AI-based detection models {Mdj}.

The modules Mdj in the bank can be chosen based on the primary purpose of the CT scan/ROI (e.g. scan for a certain disease, fractures, hemorrhage, etc.), but may also include additional modules in order to cover the possibility of incidental findings in the anatomical region covered by the scan. The output of the disease detection models Mdj is a preferably a localization of the parts of the image that causes the model to reach the finding it did, along with the certainty score *p.* For example, a high certainty score combined with no activated image region may indicate that the disease can be ruled out with high certainty.

The proposed two-stage sequential logic with condition processing for p, if q is on target (schematically "q->p") is preferred herein, in some cases the order may be reversed ("p->q"), or there is only a single stage, either *p*, *q* which is assessed, as the case may be. Thus, if the certainty check at S360 is on target, only then dose flow pass on to next step S370,

At step S370 it is then checked whether the whole of the current scan range R, (or subrange rj for second and later pass) has been analyzed at steps S330-S350. If yes, the image protocol is done at step S390, and the imagery for the covered ranges may then be output and stored possibly in association with the respective quality findings *p,q,* or otherwise processed. In particular, it is checked at S370 if the whole range R is on-target in respect of certainty (and hence IQ), and only then is the data output at S390 for the medical user to examine. Alternative, imagery that is found to be on target is immediately made available to clinical user for review or other processing.

If at step S370 it is found that a buffer filled with the off-target flags awarded at check steps S340, S360 is not empty, that is, that there are one or more sub-ranges outstanding that yielded off target imagery in respect to quality *p* and/or *q,* a re-scan is requested there at step S320, but now the IQ booster operation is applied to affect the rescan. For example, the dose profile *D(z) > D '(z)* is re-set at step S380 to a higher dose compared to dose used in the earlier pass, to so effect the IQ quality booster operation in preferred embodiments. Alternatively, or in addition, IQ booster operation may take the form of averaging over two or more images from previous two or more scan passes. The reconstructed images or their projection data frames may be added up. In the latter case, just like with the dose increasing embodiment, a new reconstruction may be done, but now based on the so combined (added or averaged), thus "new", projection data frames.

For yet better efficiency, based on the entries in the buffer, a revisit itinerary may be drawn up based on the current imaging geometry setting, such as position of table. The position that needs revisiting as per buffer, and is situated the furthest from current position, is revisited last, whilst all positions in between are revisited in turn and scanned "on the way" toward the said furthest position that needs to be revisited and rescanned.

The above is then repeated, until the whole range has been scanned at the requisite diagnostic finding certainty *p*, and hence IQ *q.* Thus, a next scan with the updated dose profile is executed. The patient table may move back and forth between the end points in a jog mode fashion to realize an efficient way of iterative scanning. Note that a narrow collimation of the cone beam of the CT scanner may be used in order to realize the full dose saving potential and selectively acquire slices. The slices reconstructed during this repeat scan are again fed into back into step S330 for reanalysis, and the protocol is repeated until certainty is achieved across the requested FOV for the regions of interest ROI.

As said, revisiting /rescans can be done until whole the initial range R is covered, in particular the ROI, and/or until a preset stopping condition (e.g., maximal amount of dose expended, max number of revisits, an allotted time limit is exhausted, etc.) is met. Such stopping condition may be needed as a stopgap. This is because, whilst the method is generally convergent to state where all of the initial range has been scanned on target (albeit at places at higher dose than others), on occasion the method may get trapped in a loop as for some reason IQ score q remains stubbornly low and/or prediction certainty p may remain low for some segments along axis Z, e.g. when they remain corrupted by repeated motion of imaged object, thus causing motion artifacts for example. In such cases, this loop deadlock situation can be brought to user's attention by whichever way, messaging, signaling etc. The user is then made aware that imaging was aborted. But still, the problematic segments may not be related to the ROI, so even in such an abort case, the original objective of imaging ROI on target can still be achieved. The problematic segments can then still be analyzed separately by using a different imaging modality or by more detailed in-depth analysis of problematic imagery *m* by a human radiologist. Thus, in such abort situations the related imagery may be flagged up accordingly so they can be picked up by radiologist in their in-tray. In such deadlock situation, when no increase in certainty score is achievable by increasing the X-ray dose to a limit predefined in the scan configuration, the corresponding slice location will not the scanned again, but the slice is flagged for inspection by a radiologist, optionally with the information which model Mdj raised the low/medium certainty score.

Thus, in either case (aborted or not), at the conclusion of the above-described protocol at step S390 there will be available a set of image data each pertaining to a different range or point on the rotation axis Z, with some or all of the underlying projection data acquired at different doses, and each associated with their respective quality *parameter p,q* optionally including an indication of the finding, in descriptive text form, as a medical code, graphically, etc. Thus, it can be seen from the manner in which the protocol flow is configured, the subranges that need rescanning and revisits will become shorter and shorter with number of passes as more and more images tend to be found compliant.

At the end of the iterative process, an overall test result may be aggregated which contains some or all findings with certainty p higher than the threshold p0. The clinical user could also be presented with an option to toggle between the various acquisitions for the rescanned c *m,* if necessary. Since dose is increased only selectively over the scan FOV, this iterative scan protocol allows "fusing" low-dose screening with diagnostic scans.

Many ways of making this scored image data (*m*(*z*)*,p,q*)_{*z*∈*Z*} available to the clinical user are envisaged herein. For example, a multi-pane "combo" graphics display is generated by the visualizer VIZ on display DD of the viewing station WS. For example, each pane may relate to a different segment z, showing the respective image m(z), possibly with an information box including the associated quality parameters *p,q.* However, in other embodiments, the graphics display may support interactive graphics user interface widgetry that may allow user to select, such as toggle, through the respective segments m(z), to view the image m(z) concerned. In another embodiment it is foremost in a panel that the imagery for the actually requested region of interest ROI is displayed, with or without scores/findings *p* and/or *q,* as this was the main purpose of the imaging. The imagery pertaining to the ROI may be found manually by user scrolling through the image volume obtained over range R, or may be located automatically by, e.g., an AI based locator model. As the imagery m(z) is now of good quality, a standard such AI model can be used, as opposed to the AI models Md that are preferably configured/trained to cope with low dose flow SNR imagery. If there are some positive subsidiary findings (that is, "condition found") this may be indicated in an information window/pane, for example in form of a counter, with zero indicating no positive subsidiary findings found, whilst and a non-zero entry is indicative that positive subsidiary finding(s) have been found. If the clinical user then so wishes, they can click or otherwise request the subsidiary image data *m* to be displayed in a separate pane, or instead of the ROI image, in the current pane. Thus, on such request, the image data pertaining to one (of the possible plural) subsidiary regions ROI' is displayed, possibly alongside the findings *p,* and optionally IQ score *q.* Any other visualization scheme of the consolidated image data sets (m(z), p, q)_{*z*∈*Z*} are also envisaged herein in embodiments.

As said, the above at Fig. 3 is mainly described by reference to the image domain embodiments, based on imagery reconstructed from the respective projection frames acquired at different doses. That is, it is such reconstructed imagery that is analyzed for *p,q,* and this is indeed envisaged herein in main embodiments. However, in other embodiments, it is the projection data itself that may be so analyzed by suitably trained machine learning models. In other embodiments, hybrid setups are contemplated, where the analysis may be based on both or either, on projection domain data and/or image domain data, as needed or as clinical user requested.

The machine learning (ML) models Mq, Md as may be used herein in steps S330, S350 may configured for classification or regression, preferably the earlier, and preferably with locator facility, thus are able to identify a region in the analyzed imagery that pertain to the predicted finding p, in case of low-dose diagnoser model Md. Discriminative probabilistic models may be used. The models Md, Mq may be arranged as convolutional neural network (CNN) models, or as fully connected models, are partly as both. Neural network (NN) type models have been found to be useful for analyzing spatial data such as is the imagery *m* to which they are applied. A corpus of historical and/or synthetically generated imagery may be used for training models Md, Mq. A training algorithm such as gradient -based ones, e.g., backpropagation algorithms, may be used. The training algorithm may be driven by an objective function that may be configured to measure a deviation between model output when applied to training input, and ground truth target data associated with the training input data. In the training, parameters of the model may be adapted to improve the objective function until a preset stopping condition is met at which point the model is considered trained and may be used for testing or deployment. A supervised, semi-supervised, self-supervised, unsupervised or reinforced training setup may be used. In order to cope with low dose, the training imagery can be annotated by human expert for medical conditions j that are present in such training imagery (e.g., historical imagery drawn from prior patients as may be held and reviewed in a PACS). The annotations may be awarded manually or by database querying or scripting or other automated way, e.g., by analyzing reports or medical records as may be held in association with the historical imagery. In a supervised scheme, the so annotated imagery can be artificially superimposed with generated noise, to so train, or later finetune, the model for coping with high noise/low SNR. For example, the annotation of the original good IQ quality image may be used as target, whilst the artificial noise corrupted image is used as training input, instead of the original/historical image used for awarding the annotation. Other low dose training strategies can be used instead. For example, GANs (Generative Adversarial Networks) may be used to generate more examples from a possible small pool of historical imagery, optionally at different noise levels, as needed. For the one or more second stage diagnostic models Mdj, respective dedicated models for a particular pathology could be developed using segmentation or object detection networks such as *UNet* type CNNs, or *Faster-RCNN*, or any other. See O Ronneberger et al in their paper "t7 Net: Convolutional Networks for Biomedical Image Segmentation" published under arXiv reference code arXiv: 1505.04597 on 18 May 2015, available online at https://arxiv.org/abs/1505.04597. *Faster-RCNN type CNNs are reported by* S Ren et al in their paper "Faster R-CNN: Towards Real-Time Object Detection with Region Proposal Network", published under arXiv reference code arXiv: 1506.01497 [cs.CV], on 04 June 2015, available online at https://arxiv.org/abs/1506.01497. The *U-net* type CNNs have a characteristic bottleneck structure with a series of data dimension reducing convolutions, followed by a series of dimension increasing deconvolutions: such ML model architectures have been found to be able to cope well with high noise data. As to the first stage IQ assessor model Mq, such are preferably, but not necessarily configured for per slice-based analysis for detection of image quality. A pre-trained deep learning model such as *EfficientNet* or related model types can be employed. See for example Mingxing Tan et al in their paper "EfficientNet: Rethinking Model Scaling for Convolutional Neural Networks", published as preprint under reference code arXiv: 1905.11946 [cs.LG] on 11 Sep 2020, available online at https://arxiv.org/abs/1905.11946v5*.* The model can then be fine-tuned using slice based expert annotations for the corresponding image quality concerns. Alternatively, object detection models such as Fast-RCNN or anomaly detection models can be alternatively used, depending on the ability to spend efforts on annotating the images. It could also be possible to train and apply such models on historical surview images from historical surview based scans, using the original surview images, or synthetically generated one from physical simulation processes, or using the said GANs, etc.

Whilst the IQ booster operation as used herein in method of the Fig. 2 was mainly described with reference to X-radiation dose increase, instead of in addition to such dose increase, the earlier mentioned averaging may be done. Thus, one or more projection data frames are voxel wises added up averaged and these are then re-reconstructed to obtain higher IQ imagery, which may then be put through the quality check. Instead of operating in the projection domain, it is the reconstructed imagery *m* that may be so added up or averaged to boost IQ.

The components of the system CLS may be implemented as one or more software modules, run on one or more general-purpose processing units PU such as a workstation associated with the imager IA, or on a server computer associated with a group of imagers.

Alternatively, some or all components of the system CLS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the system CLS may be implemented in both, partly in software and partly in hardware.

The different components of the system CLS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention, and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims indicated in brackets should not be construed as limiting the scope.

## Claims

1. System (CLS) for tomographic imaging, comprising:
an input interface (IN) for receiving a scan request for a region of interest (ROI) in a surrounding object (PAT); and
control logic (CL) operable to cause a tomographic imaging apparatus (IA) in an imaging session to:
acquire, along an acquisition axis (Z), multiple projection frames *λ*(*z*) of the surrounding object (PAT), for one or more ranges along the acquisition axis of the imaging apparatus (IA); and
whilst the imaging session is ongoing, based on a quality assessment in relation to the acquired projection frames, revisit one or more times, at least one earlier range and re-acquiring there fresh projection fames *λ'*(*z*), with application or re-application of the image quality, IQ, booster operation.

2. The system of claim 1, wherein, once the quality assessment is on target, the control logic (CL) is operable to cause no longer revisiting the relevant range for reacquisition in the current imaging session.

3. The system of claim 1 or 2, wherein the control logic (CL) to cause revisiting, one or more times, for reacquisition of fresh frames at other or the same one or more ranges, with application or re-application of the image quality, IQ, booster operation, if the quality assessment is off target, and to cause such revisiting and reacquiring other fresh frames λ'(*z*) there, with re-application of the image quality, IQ, booster operation, until the quality assessment is on target or until a stopping condition is met.

4. The system of any one of the preceding claims, wherein the quality assessment is based on respective slice imagery (*m(λ*(*z*))) reconstructable by a reconstructor (RECON) from the projection frames for the respective range along the acquisition axis.

5. The system of any one of the preceding claims, wherein the quality assessment includes at least one score that pertains to at least one of: i) an image quality, IQ, score in relation to or based on the respective projection frames per respective range on the axis, ii) a certainty score, IC, in relation to at least one finding based on the respective projection frames per range on the axis.

6. The system of claim 5, wherein the quality assessment is based on both, IQ and the IC.

7. The system of claim 6, wherein the quality assessment is two-stage, based first on IQ and then on IC, wherein the revisiting of some at least one range and reacquisition of fresh projection frames there is done only for the said at least one or more range for which the IQ is off target, or for which the IQ is on target, but the IC is not.

8. The system of any one of claims 5-7, wherein there are plural findings, and wherein one of the findings appertains to the region of interest, and at least one other finding appertains to at least one other subsidiary region (ROI') located elsewhere in respect to the axis Z from where the region of interest (ROI) is located.

9. The system of any one of the preceding claims, wherein, wherein at least one of the said ranges, on a revisit, is smaller than on at least one earlier visit.

10. The system of any one of the preceding claims, comprising the, or a, visualizer (VIZ) operable to cause displaying, or selectably so displaying, on the, or a, at least one display device (DD), of i) any one or more of various projection frames at different doses and for different ranges, or of the slice imagery reconstructable from the projection frames at different doses and for the different ranges, ii) data in relation to the respective findings including the IQ score and/or the IC score.

11. An imaging arrangement (IAR) including a system as per any one of preceding claims, and any one or more of: the imaging apparatus (IA), a display device (DD), a storage (MEM) on which is storable the projection frames and/or slice imagery reconstructable from the projection frames.

12. Method of tomographic imaging, comprising:
receiving (S310) a scan request for a region of interest (ROI) in a surrounding object (PAT);
in at least one scan of an imaging session with a tomographic imaging apparatus (IA), acquiring(S320), along an acquisition axis (*Z*) of the imaging apparatus, multiple projection frames *λ*(*z*) for some ranges along the acquisition direction for the surrounding object (PAT); and
whilst the imaging session is ongoing, based on a quality assessment in relation to the acquired projection frames, revisiting one or more times, at least one earlier range and re-acquiring (S320, S330, S360) there fresh projection fames *λ*'(*z*) with application or re-application of the image quality, IQ, booster operation.

13. The system or method according to any one of the preceding claims, wherein the image quality, IQ, booster operation includes one of i) increasing dose, and acquiring or re-acquiring at a higher dose than before, or ii) combining previously acquired frames and reconstructing same, or combining previous slice imagery.

14. A computer program element, which, when being executed by at least one computing system (CLS), is adapted to cause the computing system (CLS) to perform the method as per any one of claims 12,13.

15. At least one computer readable medium having stored thereon the program element of claim 14.
